# EUROPEAN PATENT APPLICATION

(11) **EP 0 658 340 A1**
(43) Date of publication of application: **21.06.1995**
(21) Application number: 94203477.8
(22) Date of filing: 30.11.1994
(51) Int. Cl.: A61K 7/22, A61K 7/16

(54) **Oral compositions**

(30) Priority: 17.12.1993 EP 93310260
(71) Applicant: UNILEVER N.V., NL-3000 DK Rotterdam (NL); UNILEVER PLC, London EC4P 4BQ (GB)
(72) Inventor: Hammond, Kevin, Unilever Res. Port Sunlight Lab., Bebington Wirral Merseyside L63 3JW (GB)
(74) Representative: van Gent, Jan Paulus

(57) **Abstract**

The present invention relates to oral care compositions which contain a betaine-type amphoteric surfactant. By the inclusion therein of saccharin or a saccharin alternative in conjunction with another sweetening agent such as thaumatin or stevioside, the bitter after-taste of the betaine-type amphoteric surfactant is significantly reduced.

## Description

The present invention relates to oral compositions which comprise a particular surfactant.

More particularly, the present invention relates to oral compositions which comprise a certain class of amphoteric surfactants.

Oral compositions in the context of the present invention are compositions for the care of the human teeth and mouth, and comprise compositions such as dentifrices, toothpastes, gels, mouthwashes, powders, tablets, lozenges, gargle solutions and the like.

Oral care compositions frequently contain a surfactant, and the most common class of surfactants used in oral care compositions is the class of anionic surfactants. The most frequently used surfactant of this class is sodium laurylsulphate.

This type of surfactants has, however, certain drawbacks, such as incompatibility with quaternary antibacterial compounds and biomolecules such as enzymes, bacteriophages, antibodies which may be present in the oral care compositions.

Another type of surfactants which do not show the drawbacks of sodium laurylsulphate are the amphoteric surfactants. A typical representative of this class of surfactant are the betaine-type amphoteric surfactants.

The betaine-type amphoteric surfactant can be represented by the following general formula: 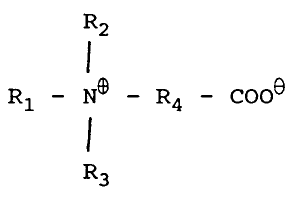
in which R₁ is a C₈-₂₀ alkyl group or the group 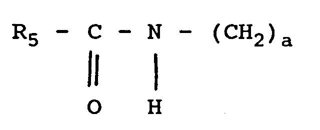
in which R₅ is a C₈-C₂₀ alkyl group and "a" ranges from 1-3, and in which R₂ and R₃ are each a C₁-C₃ alkyl group and R₄ is a C₁-C₄ alkalene group or a C₁-C₄ hydroxyalkylene group with one hydroxyl group.

Such betaine-type amphoteric surfactants have already been proposed for use in oral care compositions. Thus, for instance, in US-A-4,490,353 and 4,528,182 these surfactants have been proposed for inclusion in oral care products which comprise a quaternary ammonium compound as anti-plaque agent, e.g. benzethonium chloride.

However, we have found that brushing teeth with an oral care composition which contains a betaine-type surfactant of the above type caused a bitter-aftertaste in the mouth, as compared with similar compositions which contained sodium laurylsulphate.

Since quaternary ammonium compound-type anti-plaque agents are generally perceived to have a bitter taste, it is believed that brushing the teeth with a composition containing a mixture of the betaine-type surfactant and a quaternary ammonium compound will cause an even more pronounced bitter-aftertaste in the mouth.

We also found, that the perceived bitter-aftertaste was more pronounced with oral care compositions that contained low impact spearmint flavours than with doublemints and peppermints, but even with high impact peppermints and doublemints, still a consumer-unacceptable bitter-aftertaste was perceived.

The addition of a sweeting agent such as saccharin or a saccharine alternative did not reduce the bitter-aftertaste of these oral care compositions.

We have now surprisingly found, that the use of saccharin or a saccharin alternative selected from the groups consisting of cyclamate, aspartame, acesulfame K and chloro-sucrose in combination with another, particular sweetening agent in oral care compositions which contain the above-type betaine-type amphoteric surfactants significantly reduces the bitter-aftertaste caused by such compositions upon brushing. This particular sweetening agent is selected from the group consisting of thaumatin, stevioside, glycyrrhizin, ammonium glycyrrhizinate and neohesperidin dihydrochalcone.

Consequently, in its broadest aspect the present invention relates to an oral care composition which is substantially free from bitter-tasting quaternary ammonium compounds and which comprises a betaine-type amphoteric surfactant and saccharin or a saccharin alternative selected from the group consisting of cyclamate, aspartame, acesulfame K and chloro-sucrose, and is characterised in that the composition further comprises another sweetening agent selected from the group consisting of thaumatin, stevioside, glycyrrhizin, ammonium glycyrrhizinate and neohesperidin dihydrochalcone.

The invention will hereafter be discussed in more detail.

The betaine-type amphoteric surfactants are well-known per se.

Specific examples are:
sodium cocoamphoacetate
sodium carboxymethyl oleic polypropylamine
sodium cocoamphoproprionate
lauryl betaine
cocamidopropyl betaine
tallow dihydroxyethyl betaine
Particularly preferred are the fatty acidamidoalkyl betaines such as 1-alcoylamino-3-dimethylammonio-propane-3-carboxy methyl betaine, in which the alcoyl group contains from 7-17 carbon atoms. This product is commercially available from Th. Goldschmidt AG, Germany under the name Tego® Betain BL 281.

The amphoteric surfactant is used in the present invention in an amount of 0.01-6%, usually 0.1-3% and preferably 0.25-2% by weight.

The saccharin, either as such or as sodium saccharinate is used in an amount of 0.05-1.5, usually 0.1-1 and preferably 0.15-0.75 % by weight of the composition. The saccharine alternatives are used in the following amounts:
- Cyclamate -: used in an amount 0.3-13.0, usually 0.8-8.0 and preferably 1.0-6.0% by weight.
- Aspartame -: used in an amount 0.09-3.5, usually 0.22-2.2 and preferably 0.3-1.7% by weight.
- Acesulfame K -: used in an amount 0.14-5.25 usually 0.35-3.5 and preferably 0.45-2.5% by weight.
- Chloro-Sucrose -: used in an amount 0.02-0.7 usually 0.05-0.5 and preferably 0.07-0.35% by weight.

Of these four saccharine alternatives, Aspartame and Acesulfame K are preferred.

The particular sweetening agent selected from the group consisting of thaumatin, stevioside, glycyrrhizin, ammonium glycyrrhizinate and neohesperidin dihydrochalcone is used in low or very low levels, usually ranging from 0.001-0.2 %, preferably 0.0015-0.15 % and particularly preferably from 0.002-0.1 % by weight of the composition. The preferred sweetening agents are thaumatin and stevioside, and the above ranges particularly apply to the preferred sweetening agent thaumatin.

The oral care compositions of the present invention may furthermore comprise optional, conventional ingredients such as pharmaceutically acceptable carriers like starch, sucrose, water or water/alcohol systems etc.. Lower amounts of surfactants which are compatible with the amphoteric surfactants may also be included, such as nonionic and anionic surfactants. When formulated into a dentifrice, such formulation may contain all the usual dentifrice ingredients. Thus, they may comprise particulate abrasive materials such as silicas, aluminas, calcium carbonates, calcium pyrophosphates, dicalcium phosphates, hydroxyapatites, trimetaphosphates, insoluble hexametaphosphates and so on, usually in amounts between 5 and 60% by weight.

Furthermore, the dentifrice formulations may comprise humectants such as glycerol, sorbitol, propyleneglycol, xylitol lactitol and so on.

Binders and thickeners such as sodium carboxymethyl-cellulose, xanthan gum, gum arabic etc. may also be included, as well as synthetic polymers such as polyacrylates and carboxyvinyl polymers such as Carbopol®.

Flavours such as peppermint and spearmint oils,, including both low impact and high impact peppermints and doublemints may also be included, as well as preservatives, opacifying agents, colouring agents, pH-adjusting agents, sweetening agents and so on.

Anti-bacterial agents may also be included such as Triclosan, chlorhexidine, copper-, zinc- and stannous salts such as zinc citrate, sodium zinc citrate and stannous pyrophosphate, sanguinarine extract, metronidazole. Further examples of anti-bacterial agents are quaternary ammonium compounds such as cetylpyridinium chloride; bis-guanides such as chlorhexidine digluconate, hexetidine, octenidine, alexidine; halogenated bisphenolic compounds such as 2,2' methylenebis-(4-chloro-6-bromophenol).

Polymeric compounds which can enhance the delivery of active ingredients such as anti-bacterial agents can also be included. Examples of such polymers are copolymers of polyvinylmethylether with maleic anhydride and other similar delivery enhancing polymers, e.g. those described in DE-A-3,942,643 (Colgate)

Furthermore anti-inflammatory agents such as ibuprofen, flurbiprofen, aspirin, indomethacin etc. may also be included.

Anti-caries agents such as sodium- and stannous fluoride, aminefluorides, monosodiumfluorophosphate, casein, plaque buffers such as urea, calcium lactate, calcium glycerophosphate, strontium polyacrylates may also be included. Other optional ingredients include vitamins such as Vitamin C, plant extracts, potassium salts such as potassium citrate, potassium chloride, potassium sulphate, potassium tartrate and potassium nitrate.

Buffers and salts to buffer the pH and ionic strength of the compositions may also be included. Liposomes and other encapsulates may also be used to improve delivery or stability.

Furthermore, the oral compositions may comprise anti-calculus agents such as alkalimetal pyrophosphates, hypophosphite-containing polymers, organic phosphonates, phosphocitrates etc..

In additional, the compositions may comprise functional biomolecules such as bacteriocins, antibodies, enzymes and so on.

Other optional ingredients that may be included are e.g. bleaching agents such as peroxy compounds e.g. potassium peroxydiphosphate, effervescing systems such as sodium bicarbonate/citric acid systems, colour change systems, and so on.

When formulated as a mouthwash, the oral care composition usually comprises a water/alcohol solution, flavour, humectant, sweetener and colorant.

The present invention will further be illustrated by way of Example.

### Example

A sensory panel consisting of 20 experienced assessors evaluated the sensory properties of toothpastes having the following formulations:

| | A | B |
|---|---|---|
| Sorbitol (70%) | 45.00 | 45.00 |
| thickening silica | 10.00 | 10.00 |
| abrasive silica | 10.00 | 10.00 |
| xanthan gum | 1.00 | 1.00 |
| polyethylenenglycol (M.W. 1,500) | 5.00 | 5.00 |
| titanium dioxide | 1.00 | 1.00 |
| sodium monofluorophosphate | 0.80 | 0.80 |
| sodium laurylsulphate | 1.5 | -- |
| cocamidopropylbetaine | -- | 2.0 |
| saccharin | x | x |
| sweetening agent | y | y |
| flavour | z | z |
| water | balance | balance |

Each of the pastes to be tested were evaluated as to their sensory properties in a Qualitative Descriptor Analysis session. ½ inch of each paste was squeezed on a small square of paper, and half of this paste was taken into the mouth, and the assessor evaluated all the attributes associated with mouth effect and sensation. If desired, the assessor could retaste the remaining half of the paste to determine the flavour attributes. Between each paste evaluation, the assessors cleaned and restored the mouth with still mineral water, lime juice and dry water biscuits. The pastes were presented blind and under code. The sensory properties were ranked on a scale ranging from 0 to 70.

The following were the results concerning the bitter after-taste of the toothpastes tested:

| | A | B1 | B2 |
|---|---|---|---|
| x | 0.2 | 0.2 | 0.15 |
| y | 0 | 0 | 0.005 thaumatin |
| z | 1.2 | 1.2 | 1.2 |
| bitter after-taste | 51.8 | 66.1 | 56.5 |

| | A | B3 | B4 | B5 |
|---|---|---|---|---|
| x | 0.25 | 0.25 | 0.25 | 0.25 |
| y | 0 | 0 | 0.1 stevioside | 0.005 thaumatin |
| z | 1.1 | 1.1 | 1.1 | 1.1 |
| bitter after-taste | 38.2 | 67.1 | 61.0 | 61.4 |

## Claims

1. An oral care composition, substantially free from bitter-tasting quaternary ammonium compounds, containing a betaine-type amphoteric surfactant and saccharin or a saccharin alternative selected from the group consisting of cyclamate, aspartame, acesulfame K and chloro-sucrose, characterised in that it contains another sweetening agent selected from the group consisting of thaumatin, stevioside, glycyrrhizin, ammonium glycyrrhizinate and neohesperidin dihydrochalcone.

2. A composition according to claim 1, characterised in that the sweetening agent is thaumatin or stevioside.

3. A composition according to claim 1 or 2, characterised in that the saccharin alternative is aspartame or acesulfame K.

4. A composition according to claim 1-3, characterised in that the amphoteric surfactant is cocamidopropylbetaine.
